# EUROPÄISCHE PATENTSCHRIFT

(11) **EP 0 525 389 B1**
(45) Veröffentlichungstag und Bekanntmachung des Hinweises auf die Patenterteilung: **22.05.1996**
(21) Anmeldenummer: 92110706.6
(22) Anmeldetag: 25.06.1992
(51) Int. Cl.: A61K 9/20, A61K 9/14

(54) **Verfahren zur Herstellung mechanisch stabiler, gut zerfallender Komprimate aus kleinen wirkstoffhaltigen Formkörpern**
Process for preparing mechanically stable, well disintegrating tablets from small active agent containing solid forms
Procédé pour la préparation de comprimés mécaniquement stables et bien désintégrables à partir de petites formes dures contenant des matières actives

(30) Priorität: 04.07.1991 DE 4122217
(43) Veröffentlichungstag der Anmeldung: 03.02.1993
(73) Patentinhaber: Merz & Co. GmbH & Co., D-60318 Frankfurt (DE)
(72) Erfinder: Nürnberg, Eberhard, Prof. Dr., W-8525 Uttenreuth/Weiher (DE); Seiller, Erhard, Dr., W-6369 Nidderau 1 (DE); Kühn, Bernd, W-8520 Erlangen/Büchenbach (DE)
(74) Vertreter: Beil, Hans Christoph

(56) Entgegenhaltungen:
- EP-A- 0 255 725
- EP-A- 0 347 748
- EP-A- 0 468 247
- DATABASE WPI Week 8714, Derwent Publications Ltd., London, GB; AN 87-099083

## Beschreibung

Die Erfindung betrifft ein Verfahren zur Herstellung von Komprimaten gemäß dem Oberbegriff des Patentanspruchs 1.

Um pharmazeutische Wirkstoffe aus Arzneimittel-Konfektionierungen kontinuierlich über einen größeren Zeitraum freizusetzen, sind bereits mehrere Methoden vorgeschlagen worden. Eine solche Methode besteht darin, aus dem Wirkstoff und einem geeigneten, physiologisch akzeptablen Trägermaterial, wie beispielsweise wasserunlöslichen Kolloiden, denen auch wasserlösliche oder in Wasser quellbare Kolloide zugemischt sein können, eine Mischung herzustellen, zu Strängen mit einem Durchmesser von 0,5 bis 3 mm zu extrudieren und danach diese Stränge zu zylindrischen Formkörpern von 1 bis 3 mm Länge zu zerteilen und gegebenenfalls zu runden. Eine andere Methode besteht darin, ein physiologisch geeignetes Trägermaterial in Form von Pellets oder Kügelchen mit dem Wirkstoff zu tränken oder damit zu überziehen.

Es wurde nun gefunden, daß beim Verpressen solcher Formkörper zu Komprimaten unerwartete Schwierigkeiten auftreten. Erfolgt das Verpressen mit relativ niedriger Preßkraft, so zerfallen die Komprimate in Flüssigkeit zwar leicht wieder in die einzelnen Formkörper, sie weisen jedoch nur geringe mechanische Stabilität und Abriebfestigkeit auf und können bei der weiteren Verarbeitung, wie beispielsweise beim Abfüllen in Blisterpackungen oder beim Transport in anderen Behältnissen Beschädigungen erfahren. Wendet man beim Verpressen höhere Preßkräfte an, die zu mechanisch stabilen Komprimaten führen, so beobachtet man einen wesentlich verzögerten Zerfall, so daß das Komprimat pharmakokinetisch andere Eigenschaften als der Formkörper selbst aufweist.

Zur Verbesserung der Verpreßbarkeit von pharmazeutischen Granulaten wurde in EP-A-0 347 748 ein Überzugsverfahren für wirkstoffhaltige Granulate beschrieben, bei dem ein Polymer als Bindemittel, verbunden mit mikrokristalliner Zellulose, auf die Granulate aufgetragen wird, um eine Verpreßbarkeit der Granulate in konventionellen Tablettenmischungen zu erreichen, ohne jedoch auf einen anschließenden Zerfall in die Einzelpartikel zu achten. Gemäß der EP-A-0 255 725 wird ein Überzugsverfahren mit bestimmten Hilfsstoffen für Retardgranulate beschrieben, das einen Schutz der Granulate vor Coalgulation während des Verpressens bietet, wobei die resultierenden Tabletten auch bessere Eigenschaften aufweisen, als entsprechende Pulver- und Granulatmischungen. Bei den für mechanisch stabile Tabletten erforderlichen Preßkräften resultieren hier jedoch nicht ausreichend rasche Zerfallszeiten.

Gegenstand der Erfindung ist ein Verfahren zur Herstellung von Komprimaten aus wirkstoffhaltigen kleinen Formkörpern, bei denen diese Komprimate rasch wieder in die Formkörper zerfallen. Bei der Lösung dieser Aufgabe sollte eine Tablette gewonnen werden, die eine hinreichende Abriebfestigkeit (Friabilität) aufweist und die Freisetzungseigenschaften des Wirkstoffs aus den Formkörpern nur unwesentlich verändert. Es wurde überraschend gefunden, daß diese Aufgabe dadurch gelöst werden kann, daß die kleindimensionierten Formkörper mit einem Überzug aus folgenden Komponenten versehen werden:
- wasserlöslicher Polyvinylalkohol mit einem Estergehalt (Polyvinylacetat) von 19,4 bis 6,7 %
- Sprengmittel
- Cellulose
- gegebenenfalls weiteren Kolloiden, Hilfs-, Farb- und/oder Aromastoffen.

Diese Komponenten werden in Form einer wäßrigen oder wäßrig-oganischen Suspension, die 0,1 bis 20, vorzugsweise 4-7 Gew.-% Polyvinylalkohol, 0,1 bis 20, vorzugsweise 4-7 Gew.-% Sprengmittel, 0,1 bis 20, vorzugsweise 4-7 Gew.-% Cellulose, 0-20 Gew.-% Kolloide, 0-20 Gew.-% Hilfsstoffe, 0-5 Gew.-% Farbstoffe und/oder 0-20 Gew.-% Aromastoffe enthält, wobei die Gesamtmenge der Inhaltsstoffe der Suspension 1-40, vorzugsweise 15-25 Gew.-%, bezogen auf die gesamte Suspension beträgt, auf die Formkörper nach bekannten Verfahrensweisen aufgetragen. Der Auftrag kann beispielsweise in mehreren Schritten durch Aufsprühen in einem Dragierkessel mit zwischengeschalteten Trocknungsschritten oder auch durch kontinuierliches Besprühen und anschließendes Trocknen erfolgen. Bei der Wahl der Temperatur der Zuluft für die Trocknung ist gegebenenfalls auf die Temperaturempfindlichkeit des Wirkstoffs Rücksicht zu nehmen. Die Überzugsschicht soll so stark sein, daß sie 1-60 Gew.-%, bezogen auf die überzogenen Formkörper, beträgt.

Für die Vertragsstaaten DE, ES, FR, GB, IT wird im Hinblick auf die EPA 046 8 247 das obengenannte Verfahren, mit Ausnahme von Extrusionspeletts mit hohem Etofibratgehalt, beansprucht.

Die überzogenen Formkörper können dann nach bekannten Verfahren, beispielsweise auf einer Exzenter- oder Rundläuferpresse, zu Komprimaten mit dem gewünschten Wirkstoffgehalt verpreßt werden. Die anzuwendenden Preßkräfte liegen zwischen 2 bis 20 kN bzw. 2 bis 10 kN, vorzugsweise 2,5 bis 5 kN, insbesondere 3 bis 3,5 kN. Die erhaltenen Komprimate haben eine ausreichende Bruch- und Abriebfestigkeit und zerfallen in Wasser sehr schnell, nämlich in 5 bis 300 s, vorzugsweise 15 bis 60 s, wieder in die ursprünglichen Formkörper. Die pharmakokinetischen Eigenschaften entsprechen damit denjenigen der verwendeten Formkörper und denjenigen von Hartkapseln, die mit den gleichen Formkörpern gefüllt sind.

Charakteristisch für das erfindungsgemäße Verfahren ist, daß die auf die Formkörper aufgetragenen Überzüge nicht eine völlig glatte, sondern elektronenmikroskopisch gut erkennbar rauhe Oberfläche besitzen. Dadurch wird eine gute Verhakung der einzelnen Teilchen nach dem Verpressen zu Komprimaten mit niedrigen Preßkräften bewirkt.

Die erhaltenen Komprimate können, falls dies gewünscht wird, ihrerseits mit einem Überzug versehen werden. Dieser kann aus einer filmbildenden Substanz, gegebenenfalls unter Zusatz von Weichmachern, Gleitmitteln sowie weiteren Hilfsstoffen, wie Pigmenten, Zucker oder anderen Süßstoffen, Farb- und/oder Aromastoffen bestehen.

Als erfindungsgemäß einzusetzendes Sprengmittel kommt insbesondere quervernetztes Polyvinylpyrrolidon in Betracht. Die Cellulose wird vorzugsweise in Form von Cellulosepulver und/oder mikrokristalliner Cellulose eingesetzt. Als fakultativ verwendbare weitere Kolloide kommen insbesondere schwerlösliche Polymethylmethacrylsäureester oder Macrogol, wie beispielsweise Macrogol 6000, in Betracht.

Als Hilfsstoffe können schwerlösliche Polymersubstanzen, wie Ethylcellulose, Celluloseacetat, Polyvinylacetat oder Polymethacrylsäureester mit kationischen Ammoniumgruppen, und magensaftresistente Substanzen, wie anionische Polymethacrylsäureester, Celluloseacetphthalat oder Hydroxypropylmethylcellulosephthalat, eingesetzt werden.

Die gegebenenfalls zusätzlich einzusetzenden Farb- und Aromastoffe sind dem Fachmann bekannt. Die Auswahl bestimmter Stoffe hängt von dem gewünschten Ergebnis ab und liegt im Bereich des Wissens des Fachmanns.

### Beispiel 1

A. Es werden zunächst Extrusionspellets folgender Rezeptur hergestellt:

| | | |
|---|---|---|
| Ibuprofen | 67,2 g | 84% |
| Polymethacrylsäureester mit kationischen Ammoniumgruppen (Eudragit RS 30D), berechnet als Festsubstanz | 8,0 g | 10% |
| Triacetin | 0,8 g | 1% |
| Na-Carboxymethylcellulose | 4,0 g | 5% |
| Gesamtfeuchtgehalt | | 23% |

Die Extrusion der feuchten Mischung erfolgte in einem Walzengranulator. Die erhaltenen zylindrischen Formkörper wurden anschließend sofort ausgerundet und gut getrocknet.
B. Überzugssuspension
Eine Suspension wird unter kräftigem Rühren aus den folgenden Komponenten hergestellt:

| | |
|---|---|
| Polyvinylalkohol (Mowiol 4-88) | 4,6 % |
| Polyvinylpyrrolidon, quervernetzt (Crospovidone, Kollidon CL) | 7 % |
| Cellulosepulver (Vitacel M 80 K | 6,8 % |
| Bananenaromapulver | 1,4 % |
| Macrogol 6.000 | 0,2 % |
| Wasser | 80 % |

C. Herstellung der überzogenen Formkörper
680 g der nach A. hergestellten Formkörper werden in einem Dragierkessel in mehreren Schritten durch Aufsprühen mit einer Suspension gemäß B., deren Gehalt an Trockensubstanz 120 g beträgt, überzogen. Auf eine sorgfältige Trocknung zwischen den einzelnen Aufträgen ist zu achten.
D. Tablettierung
Aus den gemäß C. hergestellten, gut getrockneten überzogenen Formkörper werden auf einer Exzenter- oder Rundläuferpresse Komprimate mit einem Wirkstoffgehalt von 500 mg, entsprechend 700-750 mg überzogener Extrusionspellets, unter Anwendung einer Preßkraft von 3-3,5 kN verpreßt.

### Beispiel 2

A. Es werden Extrusionspelletes folgender Rezeptur hergestellt:

| | | |
|---|---|---|
| Paracetamol, feinkrist. | 70% | 350 g |
| Avicel RC 581 | 20% | 100 g |
| Avicel CL 611 | 10% | 50 g |
| Wasser, demin. | Zuschlag 35% | 173 g |

Die angefeuchtete Pulvermischung wird mittels Walzengranulator zu zylinderförmigen Pellets von 1,2 mm Durchmesser und 3-4 mm Länge verarbeitet. Diese Pellets werden im noch feuchten Zustand sofort ausgerundet und gut getrocknet.
B. Überzugssuspension
Eine Suspension wird unter kräftigem Rühren aus folgenden Komponenten hergestellt:

| | | |
|---|---|---|
| Polyvinylalkohol (Mowiol 3-83) | 4,5% | 16,6 g |
| Polyvinylpyrrolidon, quervernetzt (Polyplasdone XL) | 5,2% | 19,2 g |
| Cellulosepulver (Elcema P 050) | 5,1% | 18,9 g |
| Natriumcyclamat | 0,2% | 0,8 g |
| Wasser, demin. | 85,0% | 314,5 g |

C. Herstellung der überzogenen Formkörper
500 g der nach A. hergestellten Pellets werden in einer Wirbelschichtapparatur (Aeromatic STREA 1) durch Aufsprühen der Suspension nach B., deren Gehalt an Trockensubstanz 55,5 g beträgt (entsprechend einem Gehalt von 15% an Trockensubstanz) überzogen. Der Anteil des Überzugs an der Pelletgesamtmasse beträgt damit ca. 10 %. Anschließend werden als Endschicht 0,5% (berechnet auf die Gesamtpelletmasse) Macrogol 6000 aus wäßriger Lösung aufgesprüht.
Die erhaltenen Pellets haben einen Wirkstoffgehalt von ca. 63 %.
D. Tablettierung
Aus den gemäß C. hergestellten, gut getrockneten überzogenen Formkörpern werden auf einer Exzenter- oder Rundläuferpresse Komprimate mit einem Wirkstoffgehalt von 500 mg, entsprechend 750 bis 800 mg überzogener Extrusionspellets, mit Preßwerkzeugen 9 x 15 mm unter Anwendung einer Preßkraft von 3,0 bis 4,0 kN verpreßt.

### Beispiel 3

A. Herstellung wirkstoffhaltiger Pelletkerne
In einem Dragierkessel werden 1000 g Nonpareilles mit einem Durchmesser von 0,7 bis 0,9 mm vorgelegt und mittels Tauchrohr auf eine Temperatur von 50°C erwärmt. Auf die im Kessel rollierenden Kerne wird mit einer Zweistoffdüse eine Wirkstofflösung folgender Rezeptur eingesprüht:

| | | |
|---|---|---|
| 1-Ephedrinhydrochlorid | 24 % | 330 g |
| Wasser, demin. | 76 % | 1045 g |

Das Einsprühen der Lösung erfolgt intermittierend bei gleichzeitiger intensiver Trocknung. Es werden so Pellets mit einem Wirkstoffgehalt von 25% erhalten.
B. Überzugssuspension
Eine Suspension wird unter kräftigem Rühren aus den folgenden Komponenten hergestellt:

| | | |
|---|---|---|
| Polyvinylalkohol (Mowiol 4-88) | 6,6% | 110,0 g |
| Polyvinylpyrrolidon, quervernetzt | 6,0% | 100,0 g |
| mikrokrist. Cellulose (Avicel PH 101) | 6,0% | 100,0 g |
| Zitronenaroma | 0,98% | 16,3 g |
| Saccharin-Natrium | 0,40% | 6,7 g |
| Indigotinblau | 0,01% | 0,16g |
| L-Gelb | 0,01% | 0,16g |
| Wasser, dmin. | 80,0 % | 1333,0 g |

C. Herstellung der überzogenen Formkörper
1330 g der nach A. hergestellten wirkstoffhaltigen Pellets werden in einer Wirbelschichtapparatur (Aeromatic STREA 1 mit Wurster-Einsatz) durch Aufsprühen der Suspension nach B., deren Gehalt an Trockensubstanz 333,3 g beträgt (entsprechend einem Feststoffgehalt von 20%), überzogen. Ein Überfeuchten der Pellets ist zu vermeiden und ggf. intermittierend zu sprühen. Die Pellets werden gut nachgetrocknet und anschließend mit einer Lösung von 0,5% Macrogol 6000 (berechnet auf die Gesamtpelletmasse) in demin. Wasser besprüht.
Es werden Pellets mit einem Wirkstoffgehalt von ca. 20% und einem Überzugsanteil von 20% (berechnet jeweils auf die Gesamtmasse) erhalten.
D. Tablettierung
Aus den gemäß C. hergestellten, gut getrockneten überzogenen Formkörpern werden auf einer Exzenter- oder Rundläuferpresse Komprimate mit einem Wirkstoffgehalt von 50,0 mg, entsprechend 250-300 mg überzogener wirkstoffhaltiger Nonpareilles, mit Preßwerkzeugen von 10 mm Durchmesser unter Anwendung einer Preßkraft von 2,3 bis 3,0 kN verpreßt.

## Patentansprüche (Patentansprüche für folgende(n) Vertragsstaat(en): AT, BE, CH, DK, GR, LI, LU, MC, NL, PT, SE)

1. Verfahren zur Herstellung mechanisch stabiler, gut zerfallender Komprimate aus gegebenenfalls ausgerundeten wirkstoffhaltigen kleinen Formkörpern, dadurch gekennzeichnet, daß auf die Formkörper eine wäßrige oder wäßrig-organische Suspension, bestehend aus
a) wasserlöslichem Polyvinylalkohol mit einem Estergehalt (Polyvinylacetat) von 19,4 bis 6,7 %,
b) einem Sprengmittel,
c) Cellulose,
d) gegebenenfalls weiteren Kolloiden, Hilfsstoffen sowie Farbstoffen und/oder Aromastoffen
aufgetragen wird, die überzogenen Formkörper getrocknet und dann zu Komprimaten verpreßt werden.

2. Verfahren nach Anspruch 1, dadurch gekennzeichnet, daß als Sprengmittel quervernetztes Polyvinylpyrrolidon verwendet wird.

3. Verfahren nach einem der Ansprüche 1 oder 2, dadurch gekennzeichnet, daß die Cellulose in Form von Cellulosepulver und/oder mikrokristalliner Cellulose verwendet wird.

4. Verfahren nach einem der Ansprüche 1 bis 3, dadurch gekennzeichnet, daß die Suspension 0,1 bis 20 Gew.-% Polyvinylalkohol, 0,1-20 Gew.-% Sprengmittel, 0,1-20 Gew.-% Cellulose, 0-20 Gew.-% Aroma- und/oder Farbstoffe sowie 0-20 Gew.-% andere Kolloide und/oder andere Hilfsstoffe enthält und die Gesamtmenge der Inhaltsstoffe der Suspension 1-40, vorzugsweise 15-25 Gew.-%, bezogen auf die gesamte Suspension, beträgt.

5. Verfahren nach Anspruch 4, dadurch gekennzeichnet, daß Polyvinylalkohol, Spengmittel und Cellulose jeweils in Mengen von 4-7 Gew.-% in der Suspension enthalten sind.

6. Verfahren nach einem der Ansprüche 1 bis 5, dadurch gekennzeichnet, daß als weitere Kolloide schwerlösliche Polymethylmethacrylsäureester oder Macrogol, insbesondere Macrogol 6000, verwendet werden.

7. Verfahren nach einem der Ansprüche 1 bis 6, dadurch gekennzeichnet, daß als Hilfsstoffe magensaftresistente, filmbildende Substanzen, wie anionische Polymethacrylsäureester, Celluloseacetatphthalat oder Hydroxypropylmethylcellulosephthalat verwendet werden.

8. Verfahren nach einem der Ansprüche 1 bis 7, dadurch gekennzeichnet, daß als Hilfsstoffe schwerlösliche Polymersubstanzen, wie Ethylcellulose, Celluloseacetat, Polyvinylacetat oder Polymethacrylsäureester mit kationischen Ammoniumgruppen verwendet werden.

9. Verfahren nach einem der Ansprüche 1 bis 8, dadurch gekennzeichnet, daß der aufgebrachte Überzug der Formkörper 1 bis 60 Gew.-%, bezogen auf den überzogenen Formkörper, beträgt.

10. Verfahren nach einem der Ansprüche 1 bis 9, dadurch gekennzeichnet, daß die getrockneten Formkörper unter Anwendung eines Druckes von 2 bis 10, vorzugsweise 2,5 bis 5 und insbesondere bevorzugt 3 bis 3,5 kN, vorzugsweise auf einer Exzenter- oder Rundläuferpresse zu Komprimaten verpreßt werden.

11. Verfahren nach einem der Ansprüche 1 bis 10, dadurch gekennzeichnet, daß die Komprimate mit einem Überzug aus einer filmbildenden Substanz, die gegebenenfalls Pigmente, Zucker oder andere Süßstoffe, Farbstoff, Aromastoffe, Gleitmittel und/oder Weichmacher enthält, versehen werden.

## Patentansprüche (Patentansprüche für folgende(n) Vertragsstaat(en): DE, ES, FR, GB, IT)

1. Verfahren zur Herstellung mechanisch stabiler, gut zerfallender Komprimate, mit Ausnahme von Extrusionspellets mit hohem Etofibratgehalt, wobei die Komprimate gegebenenfalls ausgerundete wirkstoffhaltige kleine Formkörper enthalten, dadurch gekennzeichnet, daß auf die Formkörper eine wäßrige oder wäßrig-organische Suspension, bestehend aus
a) wasserlöslichem Polyvinylalkohol mit einem Estergehalt (Polyvinylacetat) von 19,4 bis 6,7 %,
b) einem Sprengmittel,
c) Cellulose,
d) gegebenenfalls weiteren Kolloiden, Hilfsstoffen sowie Farbstoffen und/oder Aromastoffen
aufgetragen wird, die überzogenen Formkörper getrocknet und dann zu Komprimaten verpreßt werden.

2. Verfahren nach Anspruch 1, dadurch gekennzeichnet, daß als Sprengmittel quervernetztes Polyvinylpyrrolidon verwendet wird.

3. Verfahren nach einem der Ansprüche 1 oder 2, dadurch gekennzeichnet, daß die Cellulose in Form von Cellulosepulver und/oder mikrokristalliner Cellulose verwendet wird.

4. Verfahren nach einem der Ansprüche 1 bis 3, dadurch gekennzeichnet, daß die Suspension 0,1 bis 20 Gew.-% Polyvinylalkohol, 0,1-20 Gew.-% Sprengmittel, 0,1-20 Gew.-% Cellulose, 0-20 Gew.-% Aroma- und/oder Farbstoffe sowie 0-20 Gew.-% andere Kolloide und/oder andere Hilfsstoffe enthält und die Gesamtmenge der Inhaltsstoffe der Suspension 1-40, vorzugsweise 15-25 Gew.-%, bezogen auf die gesamte Suspension, beträgt.

5. Verfahren nach Anspruch 4, dadurch gekennzeichnet, daß Polyvinylalkohol, Spengmittel und Cellulose jeweils in Mengen von 4-7 Gew.-% in der Suspension enthalten sind.

6. Verfahren nach einem der Ansprüche 1 bis 5, dadurch gekennzeichnet, daß als weitere Kolloide schwerlösliche Polymethylmethacrylsäureester oder Macrogol, insbesondere Macrogol 6000, verwendet werden.

7. Verfahren nach einem der Ansprüche 1 bis 6, dadurch gekennzeichnet, daß als Hilfsstoffe magensaftresistente, filmbildende Substanzen, wie anionische Polymethacrylsäureester, Celluloseacetatphthalat oder Hydroxypropylmethylcellulosephthalat verwendet werden.

8. Verfahren nach einem der Ansprüche 1 bis 7, dadurch gekennzeichnet, daß als Hilfsstoffe schwerlösliche Polymersubstanzen, wie Ethylcellulose, Celluloseacetat, Polyvinylacetat oder Polymethacrylsäureester mit kationischen Ammoniumgruppen verwendet werden.

9. Verfahren nach einem der Ansprüche 1 bis 8, dadurch gekennzeichnet, daß der aufgebrachte Überzug der Formkörper 1 bis 60 Gew.-%, bezogen auf den überzogenen Formkörper, beträgt.

10. Verfahren nach einem der Ansprüche 1 bis 9, dadurch gekennzeichnet, daß die getrockneten Formkörper unter Anwendung eines Druckes von 2 bis 10, vorzugsweise 2,5 bis 5 und insbesondere bevorzugt 3 bis 3,5 kN, vorzugsweise auf einer Exzenter- oder Rundläuferpresse zu Komprimaten verpreßt werden.

11. Verfahren nach einem der Ansprüche 1 bis 10, dadurch gekennzeichnet, daß die Komprimate mit einem Überzug aus einer filmbildenden Substanz, die gegebenenfalls Pigmente, Zucker oder andere Süßstoffe, Farbstoff, Aromastoffe, Gleitmittel und/oder Weichmacher enthält, versehen werden.

## Claims (Claims for the following Contracting State(s): AT, BE, CH, DK, GR, LI, LU, MC, NL, PT, SE)

1. Method for the preparation of mechanically stable, readily disintegrating compressed bodies consisting of optionally rounded, small shaped particles containing active ingredients, characterised in that an aqueous or aqueous-organic suspension, consisting of
a) water-soluble polyvinyl alcohol having an ester content (polyvinyl acetate) of from 19.4 to 6.7%,
b) a disintegrant,
c) cellulose,
d) optionally other colloids, auxiliary substances as well as dyes and/or flavourings
is applied to the shaped particles, the coated shaped particles are dried and then pressed to form compressed bodies.

2. Method according to claim 1, characterised in that the disintegrant used is cross-linked polyvinyl pyrrolidone.

3. Method according to one of claims 1 or 2, characterised in that the cellulose is used in the form of cellulose powder and/or microcrystalline cellulose.

4. Method according to one of claims 1 to 3, characterised in that the suspension contains 0.1 to 20 wt.% of polyvinyl alcohol, 0.1 to 20 wt.% of disintegrant, 0.1 to 20 wt.% of cellulose, 0 to 20 wt.% of flavourings and/or dyes as well as 0 to 20 wt.% of other colloids and/or other auxiliary substances and the total quantity of the constituents of the suspension is 1 to 40 wt.%, preferably 15 to 25 wt.%, referred to the total suspension.

5. Method according to claim 4, characterised in that the polyvinyl alcohol, disintegrant and cellulose are each contained in the suspension in quantities of from 4 to 7 wt.%.

6. Method according to one of claims 1 to 5, characterised in that the other colloids used are sparingly soluble polymethyl methacrylates or Macrogol, in particular Macrogol 6000.

7. Method according to one of claims 1 to 6, characterised in that the auxiliary substances used are film-forming substances which are resistant to gastric juices, such as anionic polymethacrylates, cellulose acetate phthalate or hydroxypropyl methyl cellulose phthalate.

8. Method according to one of claims 1 to 7, characterised in that the auxiliary substances used are sparingly soluble polymeric substances, such as ethyl cellulose, cellulose acetate, polyvinyl acetate or polymethacrylates having cationic ammonium groups.

9. Method according to one of claims 1 to 8, characterised in that the coating applied to the shaped particles is from 1 to 60 wt.%, referred to the coated shaped particle.

10. Method according to one of claims 1 to 9, characterised in that the dried shaped particles are pressed to form compressed bodies by the application of a pressure of from 2 to 10, preferably from 2.5 to 5 and particularly preferably from 3 to 3.5 kN, preferably on an eccentric press or on a rotary press.

11. Method according to one of claims 1 to 10, characterised in that the compressed bodies are provided with a coating consisting of a film-forming substance, which optionally contains pigments, sugar or other sweeteners, dye, flavourings, lubricants and/or plasticisers.

## Claims (Claims for the following Contracting State(s): DE, ES, FR, GB, IT)

1. Method for the preparation of mechanically stable, readily disintegrating compressed bodies, with the exception of extrusion pellets having a high etofibrate content, with the compressed bodies comprising optionally rounded, small shaped particles containing active ingredients, characterised in that an aqueous or aqueous-organic suspension, consisting of
a) water-soluble polyvinyl alcohol having an ester content (polyvinyl acetate) of from 19.4 to 6.7%,
b) a disintegrant,
c) cellulose,
d) optionally other colloids, auxiliary substances as well as dyes and/or flavourings
is applied to the shaped particles, the coated shaped particles are dried and then pressed to form compressed bodies.

2. Method according to claim 1, characterised in that the disintegrant used is cross-linked polyvinyl pyrrolidone.

3. Method according to one of claims 1 or 2, characterised in that the cellulose is used in the form of cellulose powder and/or microcrystalline cellulose.

4. Method according to one of claims 1 to 3, characterised in that the suspension contains 0.1 to 20 wt.% of polyvinyl alcohol, 0.1 to 20 wt.% of disintegrant, 0.1 to 20 wt.% of cellulose, 0 to 20 wt.% of flavourings and/or dyes as well as 0 to 20 wt.% of other colloids and/or other auxiliary substances and the total quantity of the constituents of the suspension is 1 to 40 wt.%, preferably 15 to 25 wt.%, referred to the total suspension.

5. Method according to claim 4, characterised in that the polyvinyl alcohol, disintegrant and cellulose are each contained in the suspension in quantities of from 4 to 7 wt.%.

6. Method according to one of claims 1 to 5, characterised in that the other colloids used are sparingly soluble polymethyl methacrylates or Macrogol, in particular Macrogol 6000.

7. Method according to one of claims 1 to 6, characterised in that the auxiliary substances used are film-forming substances which are resistant to gastric juices, such as anionic polymethacrylates, cellulose acetate phthalate or hydroxypropyl methyl cellulose phthalate.

8. Method according to one of claims 1 to 7, characterised in that the auxiliary substances used are sparingly soluble polymeric substances, such as ethyl cellulose, cellulose acetate, polyvinyl acetate or polymethacrylates having cationic ammonium groups.

9. Method according to one of claims 1 to 8, characterised in that the coating applied to the shaped particles is from 1 to 60 wt.%, referred to the coated shaped particle.

10. Method according to one of claims 1 to 9, characterised in that the dried shaped particles are pressed to form compressed bodies by the application of a pressure of from 2 to 10, preferably from 2.5 to 5 and particularly preferably from 3 to 3.5 kN, preferably on an eccentric press or on a rotary press.

11. Method according to one of claims 1 to 10, characterised in that the compressed bodies are provided with a coating consisting of a film-forming substance, which optionally contains pigments, sugar or other sweeteners, dye, flavourings, lubricants and/or plasticisers.

## Revendications (Revendications pour l'(les) Etat(s) contractant(s) suivant(s): AT, BE, CH, DK, GR, LI, LU, MC, NL, PT, SE)

1. Procédé pour la préparation de comprimés mécaniquement stables et bien désintégrables, constitués par de petits corps moulés contenant des matières actives, éventuellement arrondis, caractérisé en ce que, sur les corps moulés, est appliquée une suspension aqueuse ou aqueuse-organique constituée par
a) de l'alcool polyvinylique hydrosoluble possédant une teneur en ester (acétate de polyvinyle) de 19,4 à 6,7%,
b) un désintégrateur,
c) de la cellulose,
d) éventuellement d'autres colloïdes, des adjuvants, de même que des colorants et/ou des arômes,
les corps moulés enrobés sont séchés et sont ensuite transformés en comprimés.

2. Procédé selon la revendication 1, caractérisé en ce que de la polyvinylpyrrolidone ayant subi une réticulation transversale est utilisée comme désintégrateur.

3. Procédé selon l'une quelconque des revendications 1 ou 2, caractérisé en ce que la cellulose est utilisée sous la forme de poudre de cellulose et/ou de cellulose microcristalline.

4. Procédé selon l'une quelconque des revendications 1 à 3, caractérisé en ce que la suspension contient de 0,1 à 20% en poids d'alcool polyvinylique, de 0,1 à 20% en poids de désintégrateur, de 0,1 à 20% en poids de cellulose, de 0 à 20% en poids d'arômes et/ou de colorants, ainsi que de 0 à 20% en poids d'autres colloïdes et/ou d'autres adjuvants, et la quantité totale des constituants de la suspension s'élève de 1 à 40, de préférence de 15 à 25% en poids rapportés à la suspension totale.

5. Procédé selon la revendication 4, caractérisé en ce que l'alcool polyvinylique, le désintégrateur et la cellulose sont contenus respectivement dans des quantités de 4 à 7% en poids dans la suspension.

6. Procédé selon l'une quelconque des revendications 1 à 5, caractérisé en ce que des esters polyméthylméthacryliques difficilement solubles ou du Macrogol, en particulier Macrogol 6000, sont utilisés comme autres colloïdes.

7. Procédé selon l'une quelconque des revendications 1 à 6, caractérisé en ce que des substances filmogènes résistant au suc gastrique, telles que des esters polyméthacryliques anioniques, l'acétate-phtalate de cellulose ou le phtalate d'hydroxypropylméthylcellulose, sont utilisées comme adjuvants.

8. Procédé selon l'une quelconque des revendications 1 à 7, caractérisé en ce que des substances polymères difficilement solubles telles que l'éthylcellulose, l'acétate de cellulose, l'acétate de polyvinyle ou des esters polyméthacryliques contenant des groupements ammonium cationiques sont utilisées comme adjuvants.

9. Procédé selon l'une quelconque des revendications 1 à 8, caractérisé en ce que l'enrobage appliqué sur les corps moulés s'élève de 1 à 60% en poids rapportés aux corps moulés enrobés.

10. Procédé selon l'une quelconque des revendications 1 à 9, caractérisé en ce que les corps moulés séchés sont transformés en comprimés en utilisant une pression de 2 à 10, de préférence de 2,5 à 5, de manière particulièrement préférée de 3 à 3,5 kN, de préférence sur une presse à excentrique ou à table tournante.

11. Procédé selon l'une quelconque des revendications 1 à 10, caractérisé en ce que les comprimés sont munis d'un enrobage constitué d'une substance filmogène qui contient éventuellement des pigments, du sucre et d'autres édulcorants, colorants, arômes, lubrifiants et/ou plastifiants.

## Revendications (Revendications pour l'(les) Etat(s) contractant(s) suivant(s): DE, ES, FR, GB, IT)

1. Procédé pour la préparation de comprimés mécaniquement stables et bien désintégrables, à l'exception d'extrudats à teneur élevée en étofibrate, les comprimés contenant de petits corps moulés possédant une teneur en matières actives, éventuellement arrondis, caractérisé en ce que, sur les corps moulés, est appliquée une suspension aqueuse ou aqueuse-organique constituée par
a) de l'alcool polyvinylique hydrosoluble possédant une teneur en ester (acétate de polyvinyle) de 19,4 à 6,7%,
b) un désintégrateur,
c) de la cellulose,
d) éventuellement d'autres colloïdes, des adjuvants, de même que des colorants et/ou des arômes,
les corps moulés enrobés sont séchés et sont ensuite transformés en comprimés.

2. Procédé selon la revendication 1, caractérisé en ce que de la polyvinylpyrrolidone ayant subi une réticulation transversale est utilisée comme désintégrateur.

3. Procédé selon l'une quelconque des revendications 1 ou 2, caractérisé en ce que la cellulose est utilisée sous la forme de poudre de cellulose et/ou de cellulose microcristalline.

4. Procédé selon l'une quelconque des revendications 1 à 3, caractérisé en ce que la suspension contient de 0,1 à 20% en poids d'alcool polyvinylique, de 0,1 à 20% en poids de désintégrateur, de 0,1 à 20% en poids de cellulose, de 0 à 20% en poids d'arômes et/ou de colorants, ainsi que de 0 à 20% en poids d'autres colloïdes et/ou d'autres adjuvants, et la quantité totale des constituants de la suspension s'élève de 1 à 40, de préférence de 15 à 25% en poids rapportés à la suspension totale.

5. Procédé selon la revendication 4, caractérisé en ce que l'alcool polyvinylique, le désintégrateur et la cellulose sont contenus respectivement dans des quantités de 4 à 7% en poids dans la suspension.

6. Procédé selon l'une quelconque des revendications 1 à 5, caractérisé en ce que des esters polyméthylméthacryliques difficilement solubles ou du Macrogol, en particulier Macrogol 6000, sont utilisés comme autres colloïdes.

7. Procédé selon l'une quelconque des revendications 1 à 6, caractérisé en ce que des substances filmogènes résistant au suc gastrique, telles que des esters polyméthacryliques anioniques, l'acétate-phtalate de cellulose ou le phtalate d'hydroxypropylméthylcellulose, sont utilisées comme adjuvants.

8. Procédé selon l'une quelconque des revendications 1 à 7, caractérisé en ce que des substances polymères difficilement solubles telles que l'éthylcellulose, l'acétate de cellulose, l'acétate de polyvinyle ou des esters polyméthacryliques contenant des groupements ammonium cationiques sont utilisées comme adjuvants.

9. Procédé selon l'une quelconque des revendications 1 à 8, caractérisé en ce que l'enrobage appliqué sur les corps moulés s'élève de 1 à 60% en poids rapportés aux corps moulés enrobés.

10. Procédé selon l'une quelconque des revendications 1 à 9, caractérisé en ce que les corps moulés séchés sont transformés en comprimés en utilisant une pression de 2 à 10, de préférence de 2,5 à 5, de manière particulièrement préférée de 3 à 3,5 kN, de préférence sur une presse à excentrique ou à table tournante.

11. Procédé selon l'une quelconque des revendications 1 à 10, caractérisé en ce que les comprimés sont munis d'un enrobage constitué d'une substance filmogène qui contient éventuellement des pigments, du sucre et d'autres édulcorants, colorants, arômes, lubrifiants et/ou plastifiants.
